# EUROPEAN PATENT SPECIFICATION

(11) **EP 1 830 801 B1**
(45) Date of publication and mention of the grant of the patent: **15.12.2010**
(21) Application number: 05770281.3
(22) Date of filing: 10.08.2005
(51) Int. Cl.: A61K 9/20, A61K 31/4402, A61P 37/08

(54) **DROP PREPARATIONS COMPRISING DIMETINDENE**
TROPFENPRÄPARATE MIT DIMETINDEN
PRÉPARATION POUR GOUTTES COMPRENANT DU DIMÉTINDEN

(30) Priority: 11.08.2004 GB 0417909
(43) Date of publication of application: 12.09.2007
(73) Proprietor: Novartis AG, 4056 Basel (CH)
(72) Inventor: ROUSSET, Julien, CH-1630 Bulle (CH); TURIN, Eric, CH-1110 Morges (CH); STEIGER, Michel, CH-1814 La Tour-de-Peilz (CH)
(74) Representative: Crawley, Patrick Edward
(86) International application number: PCT/EP2005/008697
(87) International publication number: WO 2006/015861

(56) References cited:
- WO-A-98/00168
- WO-A-98/01134

## Description

The present invention concerns orally administerable drops comprising the pharmaceutically active substance dimetindene, which drops are characterized by advantageous properties including improved chemical and physical stability, better suitability for children and good microbiological properties.

Dimetindene is a well-known, widely used antihistaminic drug, and in the context of this document the term "dimetindene" is to be understood as including dimetindene (free base) and pharmaceutically acceptable salts thereof. In particular preferred is dimetindene maleate.

Oral drops comprising dimetindene are known in the art, e.g. as "Fenistil" drops which represent a 0.1% (w/v) solution of dimetindene maleate in water and further include more than 40% (w/v) of sorbitol, more than 5% (w/v) of ethanol, methylparaben and sodium dihydrogen phosphate as excipients [(w/v) means weight/volume].

Although said preparation is established long, it has some downsides. Its chemical and physical stability is only moderate due to the presence of high amounts of sorbitol. Said sorbitol is needed as a sweetening agent Also of concern in that respect is the presence of methylparaben, which may be subject to hydrolysis. Moreover, said preparation is not well suited to be administered to babies, toddlers and children due to its substantial content of ethanol.

It is therefore a goal of the present invention to avoid said disadvantages and provide an Improved drop preparation comprising dimetindene, which drop preparation is sorbitol-free, paraben-free, ethanol-free, and having good odor and taste.

Thus, the present invention concerns an orally administerable drop preparation which is in the form of an aqueous solution and comprises
(a) 0.01-0.5 % (w/v) dimetindene or a pharmaceutically acceptable salt thereof,
(b) 1-30 % (w/v) of propylene glycol,
   which aqueous solution is devoid of sorbitol, and
   which aqueous solution is devoid of ethanol.

Preferably, dimetindene, or a pharmaceutically acceptable salt thereof, is the only pharmaceutically active substance present in the preparation.

In the preparations of the invention, dimetindene, or a pharmaceutically acceptable salt thereof, is typically present in a concentration of 0.01-0.5 % (w/v), especially 0.02-0.3% (w/v) and in particular 0.05-0.2% (w/v).

In the preparations of the invention, the component (b) is typically present in a concentration of 1-30% (w/v), especially 3-20% (w/v) and in particular 5-15% (w/v).

In a particular embodiment, the preparations of the invention additionally comprise benzoic acid as antimicrobial preservative. In another embodiment, the preparations of the invention additionally comprise edetic acid or a salt thereof, especially disodium edetate, as chelating agent in just another embodiment, the preparations of the invention comprise both benzoic acid and disodium edetate.

If present, benzoic acid is typically used In a concentration of 0.01-0.2% (w/v), especially 0.05-0.15% (w/v). If present, edetic acid, or a salt thereof, Is typically used in a concentration of 0.005-0.2% (w/v), especially 0.02-0.16% (w/v).

In a preferred embodiment, the preparations of the invention additionally comprise saccharine sodium as sweetening agent. Moreover, the preparations of the invention optionally comprise pharmaceutically acceptable buffering agents, e.g. phosphates, citric acid or citrates. Preferred is a mixture of citric acid and phosphates.

In general, oral drop preparations are manufactured in a manner known per se.

### Example 1: Oral drops comprising 0.1% (w/v) of dimetindene maleate

### Composition

| | |
|---|---|
| Dimetindene maleate | 0.10% |
| Propylene glycol | 10.00% |
| Citric acid monohydrate | 0.50% |
| Disodium hydrogen phosphate dodecahydrate | 1.60% |
| Saccharine sodium | 0.05% |
| Disodium edetate | 0.10% |
| Benzoic acid | 0.10% |
| Purified water | to make up 100ml |

Manufacturing method: Introduce all excipients into a dissolutor and mix until complete dissolution Then add and dissolve dimetindene maleate. Filter the solution through a cartridge filter and fill into bottles with droppers.

### Comparative Example 1: Known formulation comprising 0.1 % (w/v) of dimetindene maleate

### Composition

| | |
|---|---|
| Dimetindene maleate | 0.10% |
| Sorbitol | 40-50% |
| Ethanol 96% (v/v) | 5-7% |
| Sodium dihydrogen phosphate dihydrate | 0.5-2% |
| Methylparaben | 0.05-0.3% |
| Purified water | to make up 100ml |

Comparison between Example 1 and Comparative Example 1, after storage under stress conditions:

| Property | Example 1 | Comparative Example 1 |
|---|---|---|
| Chemical stability of the antimicrobial preservative | Very good | Acceptable |
| Chemical stability of the active ingredient | Good | Acceptable |
| Discoloration during storage | Negligible | Weak |
| Change in odor | Very weak | Weak |

The advantages of Example 1, with respect to physical and chemical properties are evident.

## Claims

1. An orally administerable drop preparation which is in the form of an aqueous solution and comprises
(a) 0.01-0.5% (w/v) dimetindene or a pharmaceutically acceptable salt thereof,
(b) 1-30% (w/v) of propylene glycol,
which aqueous solution is devoid of sorbitol, and
which aqueous solution is devoid of ethanol.

2. A preparation according to claim 1, which comprises dimetindene maleate as component (a).

3. A preparation according to claim 1 or claim 2, wherein the component (a) is present in a concentration of 0.02-0.3% (w/v).

4. A preparation according to any one of claims 1-3, wherein the component (a) is present in a concentration of 0.05-0.2% (w/v).

5. A preparation according to any one of claims 1-4, wherein the component (b) is present in a concentration of 3-20% (w/v).

6. A preparation according to any one of claims 1-4, wherein the component (b) is present in a concentration of 5-15% (w/v).

7. A preparation according to any one of claims 1-6, which additionally comprises benzoic acid.

8. A preparation according to any one of claims 1-7, which additionally comprises edetic acid or a salt thereof.

9. A preparation according to any one of claims 1-8, which additionally comprises saccharine sodium.

## Patentansprüche

1. Oral verabreichbare Tropfen-Zubereitung, die in Form einer wässrigen Lösung ist und umfasst
(a) 0,01-0,5% (w/v) Dimetinden oder ein pharmazeutisch akzeptables Salz davon,
(b) 1-30% (w/v) Propylenglykol,
wobei die wässrige Lösung frei von Sorbitol ist und
wobei die wässrige Lösung frei von Ethanol ist.

2. Zubereitung nach Anspruch 1, die Dimetindenmaleat als Komponente (a) umfasst,

3. Zubereitung nach Anspruch 1 oder Anspruch 2, wobei die Komponente (a) in einer Konzentration von 0.02-0.3% (w/v) vorliegt,

4. Zubereitung nach einem der Ansprüche 1-3, wobei die Komponente (a) in einer Konzentration von 0,05-0,2% (w/v) vorliegt.

5. Zubereitung nach einem der Ansprüche 1-4, wobei die Komponente (b) in einer Konzentration von 3-20% (w/v) vorliegt

6. Zubereitung nach einem der Anspruche 1-4, wobei die Komponente (b) in einer Konzentration von 5-15% (w/v) vorliegt

7. Zubereitung nach einem der Anspruche 1-6, welche zusätzlich Benzoesäure umfaßt

8. Zubereitung nach einem der Ansprüche 1-7, welche zusätzlich Editinsäure oder ein Salz davon umfasst.

9. Zubereitung nach einem der Ansprüche 1-8, welche zusätzlich Saccharinnatrium umfasst.

## Revendications

1. Préparation pour gouttes administrable par voie orale qui se présente sous forme d'une solution aqueuse et qui comprend ;
(a) 0,01-0,5% (p/v) de dimétindène, ou d'un de ses sels pharmaceutiquement acceptables,
(b) 1-30% (p/v) de propylène glycol,
ladite solution aqueuse est dépourvue de sorbitol, et
ladite solution aqueuse est dépourvue d'éthanol.

2. Préparation selon la revendication 1, qui comprend du maléate de dimétindène en tant que composant (a).

3. Préparation selon la revendication 1 ou la revendication 2, **caractérisée en ce que** le composant (a) est présent selon une concentration allant de 0,02-0,3% (p/v).

4. Préparation selon l'une quelconque des revendications 1-3 , **caractérisée en ce que** le composant (a) est présent selon une concentration allant de 0,05-0,2% (p/v).

5. Préparation selon l'une quelconque des revendications 1-4, **caractérisée en ce que** le composant (a) est présent selon une concentration allant de 3-20% (p/v).

6. Préparation selon l'une quelconque des revendications 1-4, **caractérisée en ce que** le composant (a) est présent selon une concentration allant de 5-15% (p/v).

7. Préparation selon l'une quelconque des revendications 1-6 qui comprend en plus de l'acide benzoïque.

8. Préparation selon l'une quelconque des revendications 1 -7 qui comprend en plus de l'acide édétique ou un de ses sels.

9. Préparation selon l'une quelconque des revendications 1-8 qui comprend en plus de la saccharine de sodium.
